Europäisches Patentamt

European Patent Office

Office européen des brevets

(19)

(11) Veröffentlichungsnummer: **0 332 576 B1**

(12)

# EUROPÄISCHE PATENTSCHRIFT

(45) Veröffentlichungstag der Patentschrift: **06.04.94**

(51) Int. Cl.⁵: **C07K 7/00**, C12N 15/00, C12P 21/00, A61K 37/02

(21) Anmeldenummer: **89810152.2**

(22) Anmeldetag: **28.02.89**

(54) **Modifizierte Proteine.**

(30) Priorität: **07.03.88 CH 840/88**

(43) Veröffentlichungstag der Anmeldung:
**13.09.89 Patentblatt 89/37**

(45) Bekanntmachung des Hinweises auf die
Patenterteilung:
**06.04.94 Patentblatt 94/14**

(84) Benannte Vertragsstaaten:
**AT BE CH DE ES FR GB GR IT LI LU NL SE**

(56) Entgegenhaltungen:
**EP-A- 0 146 785**

**METHODS IN PROTEIN SEQUENCE ANALYSIS, 7th Internatl. Conference, Berlin (DE), 3.-8. Juli 1988; D.W. HEINZ et al., Seiten 415-422&NUM;**

(73) Patentinhaber: **CIBA-GEIGY AG**
**Klybeckstrasse 141**
**CH-4002 Basel(CH)**

Patentinhaber: **UCP GEN-PHARMA AG**
**Kraftstrasse 6**
**CH-8044 Zürich(CH)**

(72) Erfinder: **Grütter, Markus Gerhard, Dr.**
**Nettenbergweg**
**CH-4146 Hochwald(CH)**
Erfinder: **Heinz, Dirk**
**Wockerlestrasse 14**
**D-7888 Rheinfelden(DE)**
Erfinder: **Liersch, Manfred, Dr.**
**Rütiring 26**
**CH-4125 Riehen(CH)**

EP 0 332 576 B1

**Beschreibung**

Die Erfindung betrifft Mutanten der Protease-Inhibitoren Eglin B und Eglin C, für die Herstellung solcher Mutanten geeignete Hybridvektoren und mit solchen Hybridvektoren transformierte Wirtsmikroorganismen sowie Verfahren zur Herstellung der genannten Hybridvektoren, transformierten Wirtsmikroorganismen und Eglin-Mutanten.

In der Deutschen Offenlegungsschrift 2808396 werden zwei aus Blutegeln (Hirudo medicinalis) isolierte Protease-Inhibitoren, die als Eglin B und Eglin C bezeichnet werden, beschrieben. Diese Polypeptide bestehen aus je 70 Aminosäuren, haben ein Molekulargewicht von ca. 8100 und sind starke Inhibitoren für Chymotrypsin, Subtilisin, für die tierischen und menschlichen Granulozyten-Proteasen Elastase und Cathepsin G sowie für die Mastzellen-Protease Chymase. Dagegen werden Trypsin-ähnliche Proteasen nicht oder nur unwesentlich gehemmt.

Eglin C hat die folgende Primärstruktur:

```
H-ThrGluPheGlySerGluLeuLysSerPheProGluValValGlyLysThrVal

AspGlnAlaArgGluTyrPheThrLeuHisTyrProGlnTyrAspValTyrPheLeuProGluGly

SerProValThrLeuAspLeuArgTyrAsnArgValArgValPheTyrAsnProGlyThrAsnVal

ValAsnHisValProHisValGly-OH
```

Eglin C enthält im Gegensatz zu den meisten bekannten Protease-Inhibitoren keine Disulfid-Brücken. Es erweist sich unter Berücksichtigung seiner relativ geringen Molekülgrösse als ungewöhnlich stabil gegenüber Denaturierung durch Säure, Lauge oder Wärme und gegenüber proteolytischem Abbau. Die Primärstruktur von Eglin B unterscheidet sich von derjenigen von Eglin C durch Ersatz der Aminosäure 35, Tyrosin, durch Histidin.

Die Egline gehören zu den stärksten heute bekannten Hemmern von humaner und tierischer Granulozyten-Elastase, sowie von humanem Granulozyten-Cathepsin G. Unkontrollierte bzw. übermässige Freisetzung dieser zellulären Proteasen im Organismus kann einen Entzündungsvorgang verstärken und Gewebeabbau durch unspezifische Proteolyse hervorrufen. Diese für die intrazelluläre Verdauung zuständigen Enzyme sind im physiologischen (neutralen bis schwach alkalischen) Milieu optimal wirksam und vermögen native Gewebesubstanzen (z.B. Elastin) und humorale Faktoren (z.B. Blutgerinnungs- und Komplementfaktoren) rasch zu zerstören und zu inaktivieren. Auf Grund ihrer bis anhin bekannten Eigenschaften sind die Egline daher für die Anwendung in der medizinischen Therapie (Antiinflammation, Antiphlogistik, septischer Schock, Lungenemphysem, Mucoviscidose etc.) von grossem Interesse.

Neuerdings sind Egline durch rekombinante Gentechnik-Verfahren zugänglich geworden (vgl. Europäische Patentanmeldung Nr. 146785).

Der Erfindung liegt die Aufgabe zugrunde, ausgehend von Eglin B oder Eglin C neue Proteaseninhibitoren herzustellen.

Die Aufgabe wurde erfindungsgemäss durch die Bereitstellung von Eglin-Mutanten gelöst, die sich von den natürlichen Eglinen B und C dadurch unterscheiden, dass im Bereich des aktiven Zentrums (Aminosäuren 45 und 46, Leu-Asp) eine, zwei oder drei Aminosäuren durch andere Aminosäuren ersetzt wurden. Die erfindungsgemäss so erhaltenen Eglin-Mutanten weisen überraschende Eigenschaften auf: So zeichnen sie sich gegenüber Eglin B und Eglin C durch eine erhöhte Spezifität bei der Inhibierung bestimmter Proteasen oder durch die Inhibierung von Proteasen, welche von den Eglinen B und C nicht oder kaum inhibiert werden, beispielsweise Trypsin oder Thrombin, aus.

Insbesondere betrifft die Erfindung Eglin-Mutanten der Formel

```
R-ThrGluPheGlySerGluLeuLysSerPheProGluValValGlyLysThrVal

AspGlnAlaArgGluTyrPheThrLeuHisTyrProGlnTyrAspVal-W-PheLeuProGluGly

SerProVal-X-Y-Z-LeuArgTyrAsnArgValArgValPheTyrAsnProGlyThrAsnVal

ValAsnHisValProHisValGly-OH          (I),
```

worin R für Wasserstoff oder Acetyl steht, W Tyr oder His bedeutet, X Thr, Ser oder Pro ist, Y für Leu, Met, Arg, Lys, Phe, Tyr oder Trp steht und Z Asp, Glu, Gln, Asn, Ala, Ser oder Thr ist, mit der Massgabe, dass X von Thr verschieden ist, wenn Y für Leu steht und Z Asp ist, und Salze davon.

Die Erfindung betrifft vor allem Verbindungen der Formel I, worin R Acetyl ist, W Tyr bedeutet und entweder X Thr ist, Y Arg oder Lys ist und Z Asp, Glu, Gln, Asn, Ala, Ser oder Thr ist, oder X Pro ist, Y Met ist und Z Asp ist, oder X Thr ist, Y Phe, Tyr, Trp oder Met ist und Z Asp ist, und Salze davon.

Die Erfindung betrifft hauptsächlich Verbindungen der Formel I, worin R Acetyl ist, W Tyr bedeutet, X Thr ist, Y für Arg steht und Z Asp oder Ser ist, und Salze davon.

Insbesondere betrifft die Erfindung die Verbindung der Formel I, worin R Acetyl ist, W Tyr bedeutet, X Thr ist, Y für Arg steht und Z Asp bedeutet, und Salze davon.

Die neuen Verbindungen der Formel I können nicht nur in freier Form, sondern auch in Form ihrer Salze, insbesondere ihrer pharmazeutisch annehmbaren Salze, vorliegen. Da sie mehrere Aminosäurereste mit freien Aminogruppen bzw. Guanidinogruppen enthalten, können die erfindungsgemässen Verbindungen z.B. in Form von Säureadditionssalzen vorliegen. Als Säureadditionssalze kommen insbesondere physiologisch verträgliche Salze mit üblichen, therapeutisch anwendbaren Säuren in Betracht. Als anorganische Säuren sind die Halogenwasserstoffsäuren (wie die Chlorwasserstoffsäure), aber auch Schwefelsäure und Phosphor- bzw. Pyrophosphorsäure zu nennen; als organische Säuren sind in erster Linie Sulfonsäuren (wie die Benzol- oder p-Toluolsulfonsäure oder Niederalkansulfonsäuren, wie Methansulfonsäure) sowie Carbonsäuren, wie Essigsäure, Milchsäure, Palmitin- und Stearinsäure, Aepfelsäure, Weinsäure, Ascorbinsäure und Citronensäure, geeignet. Da die Eglin-Verbindungen auch Aminosäurereste mit freien Carboxylgruppen enthalten, können sie auch als Metallsalz, insbesondere als Alkalimetall- oder Erdalkalimetallsalz, z.B. Natrium-, Kalium-, Calcium- oder Magnesiumsalz, oder auch als Ammoniumsalz, abgeleitet von Ammoniak oder einer physiologisch verträglichen, organischen stickstoffhaltigen Base, vorliegen. Da sie aber zugleich freie Carboxylgruppen und freie Amino (und Guanidino)-Gruppen enthalten, können sie auch als inneres Salz vorliegen.

Die erfindungsgemässen Eglin-Mutanten und ihre Salze können nach an sich bekannten Verfahren, beispielsweise gentechnologischen Verfahren, hergestellt werden, z.B. indem man einen transformierten Wirtsmikroorganismus, der eine DNA enthält, welche für eine der genannten Eglin-Mutanten kodiert, kultiviert und die Eglin-Mutante oder ein Salz davon isoliert. Insbesondere werden die erfindungsgemässen Eglin-Mutanten und ihre Salze hergestellt, indem man

a. eine für die Eglin-Mutante kodierende DNA herstellt,
b. diese DNA in einen Vektor einbaut,
c. den erhaltenen Hybridvektor durch Transformation in einen Wirtsmikroorganismus einführt,
d. den transformierten Wirtsmikroorganismus unter Bedingungen kultiviert, die eine Expression der Eglin-Mutante erlauben, und
e. die Eglin-Mutante oder ein Salz davon isoliert.

## Für Eglin-Mutanten kodierende DNAs

Die Erfindung betrifft DNAs, welche eine für eine Eglin-Mutante, insbesondere für eine der oben als besonders bevorzugt genannten Eglin-Mutanten, kodierende Nucleotidsequenz aufweisen.

Die erfindungsgemässen DNAs besitzen vorzugsweise an ihren Enden flankierende, geeignete Restriktionsstellen einschliessende Sequenzen, welche den Einbau der DNA in einen Vektor ermöglichen.

Die erfindungsgemässen DNAs können nach an sich bekannten Verfahren hergestellt werden. So kann man die DNAs beispielsweise chemisch herstellen, oder man kann Fragmente davon durch chemische Synthese herstellen und diese in vorherbestimmter Weise enzymatisch verknüpfen, oder man kann eine für Eglin B oder Eglin C kodierende DNA in einem oder mehreren Schritten mutieren.

Die chemische Synthese von DNAs erfolgt nach an sich bekannten Verfahren. Geeignete Verfahrensweisen wurden von S.A. Narang [Tetrahedron 39, 3 (1983)] und in der Europäischen Patentanmeldung Nr. 146785 beschrieben.

Die Herstellung der erfindungsgemässen DNAs kann auch durch Mutation der für Eglin B oder Eglin C kodierenden DNA erfolgen. Bevorzugt wird das als "site directed mutagenesis" bekannte Verfahren angewendet [vgl. M.J. Zoller et al., Meth. Enzym. 100, 468 (1983)]. Dabei wird Eglin-Einzelstrang-DNA in den Bakteriophagen M13 kloniert, mit einem komplementären Oligonucleotid, welches das (die) die Mutation dirigierende Nucleotid (e) enthält, hybridisiert, das Hybridisierungsprodukt wird zum Doppelstrang aufgefüllt, der entstandene doppelsträngige Bakteriophage wird durch Transformation in einen geeigneten Escherichia coli-Wirt eingeführt, und nach der Kultivierung der transformierten E. coli-Zellen werden diejenigen Zellen, welche die für die Eglin-Mutante kodierende DNA enthalten, durch Hybridisierung mit dem oben genannten Oligonucleotid identifiziert.

Herstellung von Expressionsvektoren, welche eine für eine Eglin-Mutante kodierende DNA enthalten

Die Erfindung betrifft weiterhin Expressionsvektoren, welche eine für eine Eglin-Mutante kodierende DNA-Sequenz enthalten, die von einer Expressionskontrollsequenz derart reguliert wird, dass die Eglin-Mutante in einer mit diesen Expressionsvektoren transformierten Wirtszelle exprimiert wird.

Die Expressionsvektoren der vorliegenen Erfindung werden z.B. hergestellt, indem man in eine Vektor-DNA, welche eine Expressionskontrollsequenz enthält, eine für die Eglin-Mutante kodierende DNA-Sequenz derart einfügt, dass die Expressionskontrollsequenz besagte DNA-Sequenz reguliert.

Die Wahl eines geeigneten Vektors ergibt sich aus der zur Transformation vorgesehenen Wirtszelle. Geeignete Wirte sind beispielsweise Mikroorganismen, wie Hefen, z.B. Saccharomyces cerevisiae, und insbesondere Bakterienstämme, vor allem Stämme von Escherichia coli, ferner Bacillus subtilis, ferner Zellen höherer Organismen, insbesondere etablierte humane oder tierische Zellinien. Bevorzugte Wirtszellen sind Stämme von E. coli.

Grundsätzlich sind alle Vektoren geeignet, welche die erfindungsgemässen für die Eglin-Mutanten kodierenden DNA-Sequenzen in dem gewählten Wirt replizieren und exprimieren.

Beispiele für Vektoren, die zur Expression der Eglin-Mutante in einem E. coli-Stamm geeignet sind, sind Bacteriophagen, z.B. Derivate des Bacteriophagen λ, oder Plasmide, wie insbesondere das Plasmid colE1 und seine Derivate, z.B. pMB9, pSF2124, pBR317 oder pBR322. Die bevorzugten Vektoren der vorliegenen Erfindung leiten sich von Plasmid pBR322 ab. Geeignete Vektoren enthalten ein vollständiges Replicon und ein Markierungsgen, welches die Selektion und Identifizierung der mit den Expressionsplasmiden transformierten Mikroorganismen auf Grund eines phänotypischen Merkmals ermöglicht. Geeignete Markierungsgene verleihen dem Mikrooganismus beispielsweise Resistenz gegenüber Schwermetallen, Antibiotika und dergleichen. Weiterhin enthalten bevorzugte Vektoren der vorliegenden Erfindung ausserhalb der Replicon- und Markierungsgen-Regionen Erkennungssequenzen für Restriktionsendonucleasen, so dass an diesen Stellen die für die Eglin-Mutante kodierende DNA-Sequenz und gegebenenfalls die Expressions-Kontrollsequenz eingefügt werden können.

Mehrere Expressionskontrollsequenzen können zur Regulation der Expression eingesetzt werden. Insbesondere werden Expressionskontrollsequenzen stark exprimierter Gene der zu transformierenden Wirtszelle verwendet. Im Falle von pBR322 als Hybridvektor und E. coli als Wirtsmikroorganismus sind beispielsweise die Expressionskontrollsequenzen (welche unter anderem den Promotor und die ribosomale Bindungsstelle enthalten) des Lactoseoperons, Tryptophanoperons, Arabinoseoperons und dergleichen, des β-Lactamasegens, die entsprechenden Sequenzen des Phage λN-Gens oder des Phage fd-Schichtproteingens und andere geeignet. Während der Promotor des β-Lactamasegens (β-lac-Gen) bereits im Plasmid pBR322 enthalten ist, müssen die übrigen Expressionskontrollsequenzen in das Plasmid eingeführt werden. Bevorzugt als Expressionskontrollsequenz in der vorliegenden Erfindung ist diejenige des Tryptophanoperons (trp po).

Zur Replikation und Expression in Hefe geeignete Vektoren enthalten einen Hefe-Replikationsstart und einen selektiven genetischen Marker für Hefe. Hybridvektoren, die einen Hefe-Replikationsstart enthalten, z.B. das chromosomale autonom replizierende Segment (ars), bleiben nach der Transformation innerhalb der Hefezelle extrachromosomal erhalten und werden bei der Mitose autonom repliziert. Ferner können Hybridvektoren, die der Hefe-2μ-Plasmid-DNA homologe Sequenzen enthalten, verwendet werden. Solche Hybridvektoren werden durch Rekombination innerhalb der Zelle bereits vorhandenen 2μ-Plasmiden einverleibt oder replizieren autonom. Geeignete Markierungsgene für Hefe sind vor allem solche, die dem Wirt antibiotische Resistenz verleihen oder, im Fall von auxotrophen Hefemutanten, Gene, die Wirtsdefekte komplementieren. Entsprechende Gene verleihen z.B. Resistenz gegen das Antibiotikum Cycloheximid oder sorgen für Prototrophie in einer auxotrophen Hefemutante, z.B. das URA3-, LEU2-, HIS3- oder vor allem das TRP1-Gen. Vorzugsweise enthalten Hefe-Hybridvektoren weiterhin einen Replikationsstart und ein Markierungsgen für einen bakteriellen Wirt, insbesondere E. coli, damit die Konstruktion und die Klonierung der Hybridvektoren und ihrer Vorstufen in einem bakteriellen Wirt erfolgen kann. Für die Expression in Hefe geeignete Expressionskontrollsequenzen sind beispielsweise diejenigen des TRP1-, ADHI-, ADHII- oder PHO5-Gens, ferner im glykolytischen Abbau involvierte Promoter, z.B. der PGK- und der GAPDH-Promotor.

Insbesondere betrifft die Erfindung zur Replikation und phänotypischen Selektion befähigte Expressionsvektoren, welche eine Expressionskontrollsequenz und eine für die Eglin-Mutante kodierende DNA-Sequenz enthalten, wobei besagte DNA-Sequenz mitsamt Transkriptionsstartsignal und -terminationssignal sowie Translationsstartsignal und -stopsignal in besagtem Expressionsplasmid unter Regulation besagter Expressionskontrollsequenz so angeordnet ist, dass die Eglin-Mutante in einer mit besagtem Expressionsplasmid transformierten Wirtszelle exprimiert wird.

Um eine effektive Expression zu erreichen, muss das für die Eglin-Mutante kodierende Gen richtig (in "Phase") mit der Expressionskontrollsequenz angeordnet sein. Es ist vorteilhaft, die Expressionskontrollsequenz in den Bereich zwischen dem Haupt-mRNA-Start und dem ATG der Genkodiersequenz, welche natürlich mit der Expressionskontrollsequenz verknüpft ist (z.B. die β-lac-Kodiersequenz bei Verwendung des β-lac-Promotors), mit dem Eglin-Mutanten-Gen, welches vorzugsweise sein eigenes Translationsstartsignal (ATG) und Translationsstopsignal (z.B. TAG) mitbringt, zu verknüpfen. Dadurch wird eine effektive Transkription und Translation gewährleistet.

Transformation der Mikroorganismen

Die Erfindung betrifft auch ein Verfahren zur Herstellung von transformierten Wirtszellen, dadurch gekennnzeichnet, dass man eine Wirtszelle mit einem Expressionsvektor, der eine von einer Expressionskontrollsequenz regulierte, für die Eglin-Mutante kodierende DNA-Sequenz enthält, transformiert.

Geeignete Wirtszellen sind beispielsweise die oben genannten Mikroorganismen, wie Stämme von Saccharomyces cerevisiae, Bacillus subtilis und insbesondere Escherichia coli. Die Transformation mit den erfindungsgemässen Expressionsplasmiden erfolgt beispielsweise wie in der Literatur beschrieben, so für S. cerevisiae [A. Hinnen et al., Proc. Natl. Acad. Sci. USA 75, 1929 (1978)], B. subtilis [Anagnostopoulos et al., J. Bacteriol. 81, 741 (1961)] und E. coli [M. Mandel et al., J. Mol. Biol. 53, 159 (1970)]. Die Isolierung der transformierten Wirtszellen erfolgt vorteilhaft aus einem selektiven Nährmedium, dem das Biocid zugesetzt wird, gegen welches das im Expressionsplasmid enthaltene Markierungs-Gen Resistenz verleiht. Zellen, welche das Expressionsplasmid nicht enthalten, werden in einem solchen Medium abgetötet.

Die Erfindung betrifft ebenfalls die auf dem genannten Weg erhältlichen transformierten Wirtszellen.

Kultivierung der transformierten Wirtszellen und Gewinnung von Eglin-Mutanten

Die transformierten Wirtszellen werden zur Herstellung der Eglin-Mutanten verwendet. Das Verfahren zur Herstellung der Eglin-Mutanten ist dadurch gekennzeichnet, dass die oben genannten transformierten Wirtszellen kultiviert und das Produkt aus den Wirtszellen freigesetzt und isoliert wird.

Die Erfindung betrifft vor allem ein Verfahren zur Herstellung von Eglin-Mutanten der Formel I und von Salzen solcher Verbindungen, dadurch gekennzeichnet, dass mit einem Expressionsplasmid, welches eine von einer Expressionskontrollsequenz regulierte, für eine Eglin-Mutante der Formel I kodierende DNA-Sequenz enthält, transformierte Wirtszellen in einem flüssigen Nährmedium, welches assimilierbare Kohlenstoff- und Stickstoffquellen enthält, kultiviert werden und das Produkt aus den Wirtszellen freigesetzt und isoliert wird, und, falls gewünscht, ein verfahrensgemäss erhältliches Gemisch von Verbindungen der Formel I in die einzelnen Komponenten aufgetrennt wird, und, falls gewünscht, ein erhaltenes Salz in das freie Polypeptid oder ein erhaltenes Polypeptid in ein Salz desselben überführt wird.

Die Kultivierung der erfindungsgemässen transformierten Wirtszellen erfolgt in an sich bekannter Weise. So können für die Kultivierung der erfindungsgemässen, transformierten Wirtsmikroorganismen verschiedene Kohlenstoffquellen verwendet werden. Beispiele bevorzugter Kohlenstoffquellen sind assimilierbare Kohlenhydrate, wie Glucose, Maltose, Mannit oder Lactose, oder ein Acetat, das entweder allein oder in geeigneten Gemischen verwendet werden kann. Geeignete Stickstoffquellen sind beispielsweise Aminosäuren, wie Casaminosäuren, Peptide und Proteine und ihre Abbauprodukte, wie Trypton, Pepton oder Fleischextrakte; weiterhin Hefeextrakte, Malzextrakt, wie auch Ammoniumsalze, z.B. Ammoniumchlorid, -sulfat oder -nitrat, die entweder allein oder in geeigneten Mischungen verwendet werden können. Anorganische Salze, die ebenfalls verwendet werden können, sind z.B. Sulfate, Chloride, Phosphate und Carbonate von Natrium, Kalium, Magnesium und Calcium.

Weiterhin enthält das Medium z.B. wachstumsfördernde Substanzen, wie Spurenelemente, z.B. Eisen, Zink, Mangan und dergleichen, und vorzugsweise Substanzen, die einen Selektionsdruck ausüben und das Wachstum von Zellen, die das Expressionsplasmid verloren haben, verhindern. So wird dem Medium beispielsweise Ampicillin zugesetzt, wenn das Expressionsplasmid ein amp$^R$-Gen enthält. Ein solcher Zusatz von antibiotisch wirksamen Substanzen bewirkt auch, dass kontaminierende, Antibiotikaempfindliche Mikroorganismen abgetötet werden.

Die Kultivierung erfolgt nach an sich bekannten Verfahren. Die Kultivierungsbedingungen, wie Temperatur, pH-Wert des Mediums und Fermentationszeit, werden so ausgewählt, dass maximale Titer an Eglin-Mutanten erhalten werden. So wird ein E. coli- oder ein Hefe-Stamm bevorzugt unter aeroben Bedingungen in submerser Kultur unter Schütteln oder Rühren bei einer Temperatur von etwas 20 bis 40 °C, vorzugsweise etwa 30 °C, und einem pH-Wert von 4 bis 9, vorzugsweise bei pH 7, während etwa 4 bis 20 h, vorzugsweise 8 bis 12 h, kultiviert. Dabei sammelt sich das Expressionsprodukt intrazellulär an.

Wenn die Zelldichte einen ausreichenden Wert erreicht hat, wird die Kultivierung abgebrochen und das Produkt aus den Zellen des Mikroorganismus freigesetzt. Zu diesem Zweck werden die Zellen zerstört, z.B. durch Behandlung mit einem Detergens, wie SDS oder Triton, oder mit Lysozym oder einem gleichartig wirkenden Enzym lysiert. Alternativ oder zusätzlich kann man mechanische Kräfte, wie Scherkräfte (z.B. X-Presse, French-Presse, Dyno-Mill) oder Schütteln mit Glasperlen oder Aluminiumoxid, oder abwechselndes Einfrieren, z.B. in flüssigem Stickstoff, und Auftauen, z.B. auf 30° bis 40°C, sowie Ultraschall zum Aufbrechen der Zellen anwenden. Die resultierende Mischung, die Proteine, Nucleinsäuren und andere Zellbestandteile enthält, wird nach der Zentrifugation in an sich bekannter Weise an Proteinen angereichert.

So kann z.B. der grösste Teil der Nicht-Protein-Bestandteile durch Polyäthylenimin-Behandlung abgetrennt und die Proteine einschliesslich der Eglin-Verbindungen z.B. durch Sättigen der Lösung mit Ammoniumsulfat oder mit anderen Salzen ausgefällt werden. Bakterielle Proteine können auch mittels Ansäuern mit Essigsäure (z.B. 0,1 %, pH 3-5) ausgefällt werden. Eine weitere Anreicherung an Eglin-Mutanten kann durch Extraktion des essigsauren Ueberstandes mit n-Butanol erreicht werden. Weitere Reinigungsschritte umfassen bespielsweise chromatographische Verfahren, wie Ionenaustauschchromatographie, Gelfiltration, Gelpermeationschromatographie, Partitionschromatographie, HPLC, Umkehrphasen-HPLC und dergleichen. So erfolgt die Auftrennung der Mischbestandteile durch Dialyse, nach Ladung mittels Gel- oder trägerfreier Elektrophorese, nach Molekülgrösse mittels einer geeigneten Sephadex-Säule, durch Affinitätschromatographie, z.B. mit Antikörpern, insbesondere monoklonalen Antikörpern, oder mit Anhydrochymotrypsin oder Thrombin gekuppelt an einen geeigneten Träger zur Affinitätschromatographie, oder durch weitere, insbesondere aus der Literatur bekannte Verfahren.

Beispielsweise umfasst die Isolierung der exprimierten Eglin-Mutanten die folgenden Stufen: Abtrennung der Zellen aus der Kulturlösung mittels Zentrifugation, Herstellung eines Rohextrakts durch Zerstören der Zellen, z.B. mittels einer Dyno-Mill, Abzentrifugieren der unlöslichen Bestandteile; Ausfällen der bakteriellen Proteine mittels 1%iger Essigsäure, Gelfiltration an Sephadex G50 (oder G75), und gegebenenfalls Umkehrphasen-HPLC. Die Entsalzung erfolgt beispielsweise an Sephadex G25.

Zum Nachweis der Eglin-Mutanten kann der Test mit Anti-Eglin-Antikörpern, wie aus Kaninchen erhältlichen polyklonalen Antikörpern oder aus Hybridomazellen erhältlichen monoklonalen Antikörpern, z.B. die von Hybridoma-Zellinien 299S18-20 (CNCM I-361), 299S20-1 (CNCM I-362) oder 299S20-10 (CNCM I-363) ausgeschiedenen monoklonalen Antikörper, oder die Hemmung der Zielproteasen, z.B. Humanleukozyten-Elastase (HLE) oder Cathepsin G (Cat G) [vgl. U. Seemüller et al., Hoppe-Seyler's Z. physiol. Chem. 358, 1105 (1977); U. Seemüller et al., Meth. Enzym. 80, 804 (1981)] herangezogen werden.

Ein verfahrensgemäss erhältliches Gemisch von Verbindungen der Formel I, beispielsweise bestehend aus Verbindungen der Formel I, worin R entweder H oder Acetyl bedeutet, kann in an sich bekannter Weise in die einzelnen Komponenten aufgetrennt werden. Geeignete Trennverfahren sind beispielsweise chromatographische Verfahren, z.B. Adsorption-Chromatographie, Ionenaustauschchromatographie, HPLC oder reversed-phase HPLC, ferner multiplikative Verteilung oder elektrophoretische Methoden, z.B. Elektrophorese an Celluloseacetat oder Gelelektrophorese, insbesondere Polyacrylamid-Gelelektrophorese ("PAGE").

Je nach Arbeitsweise erhält man die erfindungsgemässen Verbindungen in freier Form oder in Form von Säureadditionssalzen, inneren Salzen oder Salzen mit Basen. Aus den Säureadditionssalzen können in an sich bekannter Weise die freien Verbindungen gewonnen werden. Von letzteren wiederum lassen sich durch Umsetzen mit Säuren oder Basen, z.B. mit solchen, die die obengenannten Salze bilden, und Eindampfen oder Lyophilisieren therapeutisch annehmbare Säureadditionssalze oder Metallsalze gewinnen. Die inneren Salze können durch Einstellen des pH auf einen geeigneten Neutralpunkt gewonnen werden.

## Pharmazeutische Präparate

Die neuen Eglin-Mutanten der Formel I weisen wertvolle pharmakologische Eigenschaften auf und können bei der Prophylaxe oder Therapie von Krankheitszuständen, die den Einsatz von Protease-Inhibitoren erfordern, angewendet werden.

Die neuen Eglin-Mutanten weisen als Protease-Inhibitoren ein Wirkungsprofil auf, welches sich von demjenigen der natürlichen Egline B und C dadurch unterscheidet, dass die Inhibitionswirkung gegenüber bestimmten Proteasen verstärkt wird bzw. bestimmte von den natürlichen Eglinen nicht inhibierte Proteasen stark inhibiert werden, während die Inhibitionswirkung gegenüber einigen natürlichen Zielproteasen der Egline, wie z.B. Granulozyten-Elastase, abgeschwächt wird oder verschwindet. So weisen beispielsweise Verbindungen der Formel I, worin R Wasserstoff oder Acetyl ist, W Tyr oder His bedeutet, X Pro ist, Y für Met steht und Z Asp ist, eine gegenüber den natürlichen Eglinen verstärkte Inhibition der humanen und tierischen Granulozyten-Elastase auf und können dementsprechend wie die natürlichen Egline z.B. bei der Behandlung von Lungenemphysem, ARDS ("acute respiratory distress syndrome"), septischem Schock, rheumatischer Arthritis und Mucoviscidose Verwendung finden Verbindungen der Formel I, worin R und W die unter Formel I angegebenen Bedeutungen haben, X Thr ist, Y Arg oder Lys bedeutet und Z Asp ist, besitzen im Gegensatz zu den natürlichen Eglinen eine ausgeprägte Inhibitionswirkung auf Trypsin und nur noch eine geringe Wirkung gegenüber Granulozyten-Elastase und Cathepsin G. Dies trifft insbesondere auf die Verbindung der Formel I zu, worin R Acetyl ist, W Tyr bedeutet, X Thr ist, Y für Arg steht und Z Asp bedeutet. Solche Verbindungen können, in Analogie zu Aprotinin, z.B. zur Behandlung der Pankreatitis und des traumatischen, pankreatogenen und hämorrhagischen Schocks eingesetzt werden. Verbindungen der Formel I, worin R, W und X die unter Formel I angegebenen Bedeutungen haben, Y Arg oder Lys ist und Z für Asp steht, weisen eine ausgeprägte Inhibitionswirkung gegenüber Thrombin auf und können, vorzugsweise in Kombination mit Antithrombin-III, z.B. zur Behandlung von Thrombosen, Thromboembolien, septischem und posttraumatischem Schock und Verbrauchskoagulopathien verwendet werden.

Die Erfindung betrifft ebenfalls pharmazeutische Zusammensetzungen, welche wenigstens eine der erfindungsgemässen Verbindungen oder deren pharmazeutisch annehmbaren Salze, gegebenenfalls zusammen mit einem konventionellen pharmazeutisch annehmbaren Träger und/oder Hilfsstoffen, enthalten.

Diese Zusammensetzungen können insbesondere bei den oben angegebenen Indikationen Verwendung finden, wenn sie z.B. parenteral, wie intravenös, intracutan, subcutan oder intramuskulär, oder topisch verabreicht werden.

Die Erfindung betrifft ebenfalls die Verwendung der erfindungsgemässen neuen Verbindungen und diese enthaltende pharmazeutische Zusammensetzungen für die prophylaktische und therapeutische Behandlung des menschlichen und tierischen Körpers, insbesondere bei den oben angegebenen Krankheitsbildern.

Die Dosierung hängt in erster Linie von der spezifischen Verabreichungsform und vom Zweck der Therapie bzw. Prophylaxe ab. Die Grösse der Einzeldosen sowie das Verabreichungsschema kann am besten anhand einer individuellen Beurteilung des jeweiligen Krankheitsfalles bestimmt werden; die dazu erforderlichen Methoden zur Bestimmung von relevanten Faktoren, wie Blutfaktoren, sind dem Fachmann geläufig.

Im Falle von Thrombin-inhibierenden Eglin-Mutanten liegt bei einer Injektion die therapeutisch wirksame Menge im Dosisbereich von etwa 0,005 bis etwa 0,1 mg/kg Körpergewicht. Bevorzugt ist der Bereich von etwa 0,01 bis etwa 0,05 mg/kg Körpergewicht. Die Verabreichung erfolgt durch intravenöse, intramuskuläre oder subcutane Injektion. Dementsprechend enthalten pharmazeutische Präparate zur parenteralen Verabreichung in Einzeldosis-Form in Abhängigkeit von der Applikationsart pro Dosis etwa 0,4 bis etwa 7,5 mg der erfindungsgemässen Verbindung. Neben dem Wirkstoff enthalten diese pharmazeutischen Zusammensetzungen üblicherweise noch einen Puffer, z.B. einen Phosphatpuffer, der den pH-Wert zwischen etwa 3,5 und 7 halten soll, und ferner Natriumchlorid, Mannit oder Sorbit zur Einstellung der Isotonie. Sie können in gefriergetrockneter oder gelöster Form vorliegen, wobei Lösungen mit Vorteil ein antibakteriell wirkendes Konservierungsmittel, z.B. 0,2 bis 0,3 % 4-Hydroxybenzoesäuremethylesteroder -ethylester, enthalten können.

Ein Präparat für die topische Anwendung von Thrombin-inhibierenden Eglin-Mutanten kann als wässrige Lösung, Lotion oder Gelee, ölige Lösung oder Suspension, oder fetthaltige oder insbesondere Emulsions-Salbe vorliegen. Ein Präparat in Form einer wässrigen Lösung erhält man beispielsweise dadurch, dass man die erfindungsgemässen Wirkstoffe oder ein therapeutisch annehmbares Salz davon in einer wässrigen Pufferlösung von pH 4 bis 6,5 löst und gewünschtenfalls einen weiteren Wirkstoff, z.B. ein Antiinflammatorikum, und/oder ein polymeres Haftmittel, z.B. Polyvinylpyrrolidon, und/oder ein Konservierungsmittel zufügt. Die Konzentration des Wirkstoffs beträgt etwa 0,1 bis etwa 1,5 mg, vorzugsweise 0,25 bis 1,0 mg, in 10 ml einer Lösung bzw. 10 g eines Gelees. Eine ölige Applikationsform für die topische Verabreichung erhält man beispielsweise durch Suspendieren der erfindungsgemässen Wirkstoffe oder eines therapeutisch annehmbaren Salzes davon in einem Oel, gegebenenfalls unter Zusatz von Quellmitteln, wie Aluminiumstearat, und/oder grenzflächenaktiven Mitteln (Tensiden), deren HLB-Wert ("hydrophilic-lipophilic-balance") unter 10 liegt, wie Fettsäuremonoester mehrwertiger Alkohole, z.B. Glycerinmonostearat, Sorbitanmonolaurat, Sorbitanmonostearat oder Sorbitanmonooleat. Eine fetthaltige Salbe erhält man z.B. durch Suspendieren der erfindungsgemässen Wirkstoffe oder deren Salze in einer streichbaren Fettgrundlage, gegebenenfalls unter Zusatz eines Tensids vom HLB-Wert unter 10. Eine Emulsionssalbe erhält man durch Verreiben einer wässrigen Lösung der erfindungsgemässen Wirkstoffe oder deren Salze in einer weichen, streichbaren Fettunterlage unter Zusatz eines Tensids, dessen HLB-Wert unter 10 liegt. Alle diese topischen Applikationsformen können auch Konservierungsmittel enthalten. Die Konzentration des Wirkstoffes beträgt etwa 0,1 bis etwa 1,5 mg, vorzugsweise 0,25 bis 1,0 mg, in etwa 10 g der Grundmasse.

Die Verabreichung von Elastase-inhibierenden Eglin-Mutanten erfolgt durch intravenöse Injektion oder intrapulmonal, durch Inhalation, z.B. mit einem Bird-Gerät. Dementsprechend enthalten pharmazeutische Präparate zur parenteralen Verabreichung in Einzeldosis-Form in Abhängigkeit von der Applikationsart pro Dosis etwa 10 bis 50 mg der erfindungsgemässen Verbindungen. Neben dem Wirkstoff enthalten diese pharmazeutischen Zusammensetzungen üblicherweise noch Natriumchlorid, Mannit oder Sorbit zur Einstellung der Isotonie. Sie können in gefriergetrockneter oder gelöster Form vorliegen, wobei Lösungen mit Vorteil ein antibakteriell wirkendes Konservierungsmittel, z.B. 0,2 bis 0,3 % 4-Hydroxybenzoesäure-methylester oder -ethylester, enthalten können.

Präparate für die topische Anwendung von Elastase-inhibierenden Eglin-Mutanten entsprechen weitgehend den oben beschriebenen.

Inhalationspräparate für die Behandlung der Atemwege durch intrapulmonale Verabreichung sind z.B. Aerosole oder Sprays, welche den pharmakologischen Wirkstoff in Form von Tropfen einer Lösung oder Suspension verteilen können. Präparate, in welchen der pharmakologische Wirkstoff in Lösung vorliegt, enthalten ausser diesem ein geeignetes Treibmittel, ferner, falls notwendig, ein zusätzliches Lösungsmittel und/oder einen Stabilisator. Anstelle des Treibgases kann auch Druckluft verwendet werden, wobei diese mittels einer geeigneten Verdichtungs- und Entspannungsvorrichtung nach Bedarf erzeugt werden kann. Besonders geeignet für die Verabreichung sind Bird-Beatmungsgeräte, welche in der Medizin eingeführt und bekannt sind; dabei wird eine Lösung des Wirkstoffs ins Gerät gegeben und mit leichtem Ueberdruck vernebelt und in die Lunge des beatmeten Patienten gebracht.

Bei Elastase-inhibierenden Eglin-Mutanten beträgt die Dosierung je nach Alter, individuellem Zustand und Art der Erkrankung bei einem Warmblüter (Mensch oder Tier) von etwa 70 kg Gewicht bei intrapulmonaler Verabreichung etwa 10 bis etwa 30 mg pro Inhalation (ein- bis zweimal täglich) und bei intravenöser Verabreichung, z.B. auch durch Dauerinfusion, bei etwa 10 bis etwa 1000 mg pro Tag. Therapeutisch wirksame Sputum- und Plasmakonzentrationen, welche mittels immunologischer Verfahren, wie ELISA, bestimmt werden können, liegen zwischen 10 und 100 $\mu$g/ml (ca. 1 bis 10 $\mu$Mol/l).

Die Verabreichung von Trypsin-inhibierenden Eglin-Mutanten erfolgt z.B. durch parenterale, wie intravenöse, intramuskuläre oder subcutane, Injektion. Die therapeutisch wirksame Menge liegt im Dosisbereich von etwa 1 bis 20 mg Wirkstoff/kg Körpergewicht. Die pharmazeutischen Präparate enthalten den Wirkstoff in einer Konzentration von ca. 0,1 bis ca. 100 mg/ml Lösung. Neben dem Wirkstoff enthalten diese pharmazeutischen Präparate noch einen Puffer, z.B. einen Phosphatpuffer (siehe oben), sowie Natriumchlorid, Mannit oder Sorbit zur Einstellung der Isotonie.

Schädlingsbekämpfungsmittel

Die neuen Eglin-Mutanten der Formel I können auch als Proteinase-Inhibitoren in der Schädlingsbekämpfung bei Pflanzen Einsatz finden. Von den natürlicherweise in verschiedenen Pflanzenarten vorkommenden Serinprotease-Inhibitoren wird angenommen, dass sie einen effizienten Schutz gegen phytopathogene Insekten, Pilze und andere Mikroorganismen darstellen, indem sie die Serinproteasen in den genannten Organismen hemmen. Darüber hinaus können sowohl monokotyle als auch dikotyle Pflanzen mit einer DNA, die für einen heterologen Protease-Inhibitor wie z.B. für Eglin B oder C oder für eine Eglin-Mutante der Formel I kodiert, transformiert werden. Die Expression der entsprechenden aktiven Proteinase-Inhibitoren verleiht den transformierten Pflanzen Schutz gegenüber dem Befall mit Phytopathogenen.

Zur Transformation von Pflanzen mit geeigneten Expressionsvektoren, die eine für eine erfindungsgemässe Verbindung kodierende DNA und Expressions-Kontrollsequenzen enthalten, stehen an sich bekannte Methoden zur Verfügung wie z.B. die Kokultivierung von Protoplasten oder isolierten Gewebestücken mit Agrobakterien, welche die entsprechenden Vektoren enthalten und der anschliessenden Regeneration zu kompletten Pflanzen oder die Uebertragung von Vektoren mit Hilfe von geeigneten, gegebenenfalls modifizierten Viren, wie z.B. TMV (Tabak Mosaik Virus) oder CaMV ("cauliflower mosaic virus"). Weitere Methoden sind z.B. die direkte Uebertragung von isolierter DNA (bevorzugt ein Fall von monokotylen Pflanzen wie Mais, Hafer, Gerste, Reis, Sorghum, Weizen, Zuckerrohr und anderen) mit Hilfe von PEG, durch Elektroporation, durch Mikroinjektion von DNA in isolierte Protoplasten, Pflanzenkalli oder Embryonen oder durch "microprojectile bombardment" und andere.

Zur Transformation in Pflanzen eignen sich DNA-Moleküle, die für eine der erfindungsgemässen Verbindungen kodieren. Bevorzugt sind solche DNA-Moleküle, die für eine Eglin-Mutante der Formel I, worin R Acetyl ist, W Tyr bedeutet, X Thr ist, Y für Arg steht und Z Asp bedeutet, kodieren. Die protease-inhibierende Wirkung der entsprechenden bevorzugten Eglin-Mutante kann in Versuchen mit dem Maispathogen Diabrotica virgifera ("western corn root worm") getestet werden. Zusatz der bevorzugten Eglin-Mutante zu Homogenaten aus Darmgewebe des Pathogens führt zu einer starken Hemmung der proteolyti-

8

schen Aktivität.

Die Erfindung betrifft insbesondere die in den Beispielen beschriebenen Eglin-Mutanten, die dafür kodierenden DNAs, solche DNAs enthaltende Expressionsplasmide, solche Expressionsplasmide enthaltende Wirtsmikroorganismen und die beschriebenen Verfahren zu deren Herstellung.

Die folgenden Beispiele dienen zur Illustration der Erfindung und sollen sie in keiner Weise einschränken.

Experimenteller Teil

Beispiel 1: M13 Klonierung des Eglin C-Gens

Aus 10 $\mu$g des Plasmids pML 147 werden durch Verdauung mit den Restriktions-Endonucleasen EcoRI und BamHI und anschliessende Elektrophorese in 1,5 % niedrigschmelzender Agarose ca. 0,5 $\mu$g des 230 bp grossen EcoRI-BamHI-Fragmentes (enthält das vollständige Eglin C-Gen) isoliert. Dieses DNA-Fragment (10 ng) wird mit 40 ng M13mp8 DNA (vorverdaut mit EcoRI und BamHI) gemischt und in 50 mM Tris-HCl pH 7,4, 10 mM MgCl$_2$, 10 mM ATP, 10 mM Dithiothreitol in Anwesenheit von 0,125 Einheiten T$_4$-DNA-Ligase in einem Volumen von 15 $\mu$l inkubiert [Zoller et al., Methods Enzym. 100, 468-500 (1983)]. Die entstandene Lösung wird zur Transformation des E. coli-Stammes JM101 (Zoller et al, s.o.) verwendet.

Die Transformationsmischung wird auf X-Gal (IPTG-Indikator)-Agar Platten ausgestrichen (Zoller et al., s.o.). Man erhält 40 blaue (Wildtyp) und 650 farblose Plaques.

Beispiel 2: Herstellung von M13mp8 Einzelstrang-DNA

2 ml einer Kultur von E. coli JM101 (gewachsen in L-Medium: 10 g Bacto-Tryptone, 5 g Bacto-Hefeextrakt, 5 g NaCl, 5 g Glucose, 0,1 g Ampicillin pro 1 l, bis zu einer OD$_{623}$ = ca. 0,5) werden mit einem farblosen Plaque (gepickt von der Agar Platte, siehe Beispiel 1) überimpft und ca. 4-5 Stunden bei 37°C, 180 upm gehalten. Anschliessend wird die gewachsene Kultur 5 Minuten in einer Eppendorf-Zentrifuge zentrifugiert. Der Ueberstand wird in ein frisches Zentrifugenröhrchen überführt, nochmals zentrifugiert und mit 200 $\mu$l 20 % Polyäthylenglykol, 1,5 M NaCl versetzt, 20 Minuten bei Raumtemperatur gehalten und anschliessend erneut zentrifugiert. Der Ueberstand wird verworfen und das Pellet in 100 $\mu$l 50 mM Tris-HCl pH 7,8, 1 mM EDTA (TE) aufgelöst. Die Mischung wird mit 50 $\mu$l Phenol/TE gemischt (15 Minuten bei Raumtemperatur) und dann 5 Minuten in der Eppendorf-Zentrifuge zentrifugiert. 100 $\mu$l vom Ueberstand werden mit 10 $\mu$l Natriumacetat pH 6 und 3 Volumen absolutem Ethanol (250 $\mu$l) versetzt, über Nacht bei -20°C gehalten und dann 10 Minuten wie oben zentrifugiert. Das Pellet wird mit 1 ml 80 % Ethanol gewaschen und erneut zentrifugiert. Das Pellet wird 10 Minuten bei Raumtemperatur getrocknet und dann in 50 $\mu$l TE gelöst. Die Lösung enthält ca. 5 $\mu$g M13 mp8 Einzelstrang DNA.

Beispiel 3: Herstellung des für [Arg45]-Eglin C kodierenden Gens

a. Kinasierung des mutagenen Oligonucleotids

Für die Mutagenese wird durch chemische Synthese folgendes Oligonucleotid hergestellt:

$$\overset{*}{5'-dCTCCTTGTTACTCGGGACC-3'}$$

10 $\mu$l des Oligonucleotids (1 OD/ml = 500 ng) werden in 20 $\mu$l 0,07 M Tris-HCl pH 7,6, 0,01 M MgCl$_2$, 50 mM Dithiothreitol mit [$\gamma$-$^{32}$P]ATP und T$_4$ Polynucleotid-Kinase (Boehringer) kinasiert [vgl. Molecular Cloning, A Laboratory Manual, ed. T. Maniatis et al., S. 125] Das kinasierte Oligonucleotid wird in 10 $\mu$l TE (50 ng/$\mu$l) gelöst.

b. <u>Mutation des Eglin C Gens</u>

```
        3'-GA GGA CAA TGA GAC CTG G-5'

                              *
   +    5'-CT CCT GTT ACT CGG GAC C-3'              (I)
        (Oligo-primer)


                              *
        5'-CT CCT GTT ACT CGG GAC C-3'
                                                   (II)
        3'-GA GGA CAA TGA GAC CTG G-5'

          │ Mutation
          ▼
          ⁴²Pro Val Thr Arg Asp
        5'-CT CCT GTT ACT CGG GAC C-3'
                                                   (III)
        3'-GA GGA CAA TGA GCC CTG G-5'
```

<u>SCHEMA DER MUTATION</u>

1 μg der M13mp8 Einzelstrang-DNA wird mit 50 ng des kinasierten Oligonucleotid-Primers in 10 μl 50 mM Tris-HCl pH 7,8, 100 mM $MgCl_2$ bei 45°C (30 Minuten) und dann bei Raumtemperatur (5 Minuten) gehalten ("annealing"). Dann werden zu dieser Mischung gegeben:
je 1 μl 10 mM dATP, dGTP, dCTP, dTTP,

 1 μl $T_4$-DNA-Ligase

 2 μl 50 mM Dithiothreitol

 1 μl 10 mM ATP

 1 μl 5 mg/ml Gelatine

 1 μl 10 x konz. Klenow-Puffer (0,66 M Tris-HCl pH 7,6, 50 mM $MgCl_2$, 50 mM Dithiothreitol)

 1 μl DNA-Polymerase (Klenow Fragment) = 2,5 Einheiten

Die Mischung wird 5 Minuten bei 22°C und dann 16 Stunden bei 15°C gehalten und schliesslich in 1 % Agarose elektrophoretisch aufgetrennt. Die entstandene zirkuläre, doppelsträngige DNA wird mit Ethidium-bromid sichtbar gemacht und aus dem Gel durch Elektroelution isoliert (ca. 10 ng in 15 μl TE).

5 μl (= ca. 3,5 ng) der auf diese Weise gewonnenen DNA werden in den <u>E. coli</u>-Stamm JM101 transformiert und auf X-Gal/IPTG Indikatorplatten ausgestrichen (siehe Beispiel 1). Ca. 100 farblose Plaques werden erhalten.

40 dieser Plaques werden zur Animpfung einer 2 ml <u>E. coli</u> JM101-Kultur verwendet (siehe Beispiel 2). Die <u>E. coli</u> Zellen werden nach der Kultivierung (Beispiel 2) vom Ueberstand (enthält Phagen und Einzelstrang-DNA, das Zellpellet enthält bereits die entsprechende, mutierte Doppelstrang-DNA) abzentrifugiert.

Je 50 μl der 40 Phagen-Ueberstände werden über Nitrozellulose filtriert, gewaschen (2 x TE), 2 Stunden bei 80°C im Vakuum gehalten und nach Southern [J. Mol. Biol. <u>98</u>, 503-517 (1975)] mit dem Oligonucleotid-Primer als radioaktiver Sonde auf das Vorhandensein der mutierten DNA-Sequenz (III, siehe Schema) geprüft (Hybridisierung). Daraus resultieren 12 potentielle, das [Arg45]-Eglin C-Gen enthaltende Phagenüberstände.

Vier dieser positiven Phagenüberstände werden verdünnt (ca. $1:10^5$), mit <u>E. coli</u> Stamm JM101 gemischt und auf Indikator-Agar ausgestrichen. Phagen aus je drei der entstandenen Plaques werden isoliert. Wie oben beschrieben wird daraus die Einzelstrang-DNA isoliert. Diese 12 Einzelstrang-DNAs werden nach Sanger [Science <u>214</u>, 1205 (1981); Proc. Natl. Acad. Sci. USA <u>74</u>, 5463 (1977)] sequenziert. Alle 12 Einzelstrang-DNAs zeigen die gewünschte, mutierte Eglin C-Sequenz.

Aus den entsprechenden <u>E. coli</u>-Zellpellets (siehe oben) wird daraufhin in einer Minipräparation die betreffende, mutierte Doppelstrang-DNA ([Arg45]-Eglin C-Gen im Plasmid M13mp8) hergestellt.

Durch Restriktions-Verdau mit den Restriktionsendonucleasen EcoRI und BamHI wird das das mutierte Gen enthaltene EcoRI-BamHI-Insert aus dem Vektor herausgeschnitten, isoliert und in den Vektor pHRi148/EcoRI/BamHI (Europäische Patentanmeldung Nr. 146785) kloniert. Das daraus entstandene Plasmid pJB618 wird isoliert und in den <u>E. coli</u>-Stamm HB101 transformiert. Der mit dem Plasmid pJB618 transformierte Stamm wird als <u>E. coli</u> HB101/pJB618 bezeichnet.

Beispiel 4: Herstellung des für [Pro44]-Eglin C kodierenden Gens

In analoger Weise wie in Beispiel 3 beschrieben wird die [Thr44]→[Pro44]-Mutation durchgeführt. Das verwendete mutagene Oligonucleotid hat folgende Struktur

$$
\overset{*}{\phantom{.}}
$$

5'-CT CCT GTT ĊCT CTG GAC-3'

Die Mutation des Eglin C Gens ist im folgenden Schema dargestellt.

```
            3'-GA GGA CAA TGA GAC CTG-5'
                              *                        (I)
        +   5'-CT CCT GTT ĊCT CTG GAC-3'
            (Oligo-primer)


            5'-CT CCT GTT ĊCT CTG GAC-3'
                              *                        (II)
            3'-GA GGA CAA TGA GAC CTG-5'

              │ Mutation

              ⁴²Pro  Val Pro Leu Asp
            5'-CT CCT GTT CCT CTG GAC-3'
                                                       (III)
            3'-GA GGA CAA GGA GAC CTG-5'
```

Bei der Aufarbeitung des Mutationsgemisches werden 18 potentielle [Pro44]-Eglin C-Mutanten erhalten. Durch Klonierung der [Pro44]-Eglin C-DNA in den Vektor pHRi148/EcoRI/BamHI erhält man in analoger Weise, wie in Beispiel 3 beschrieben, das Plasmid pJB591. Der mit diesem Plasmid transformierte E. coli HB101-Stamm wird als E. coli HB101/pJB591 bezeichnet.

Beispiel 5: Herstellung des für [Arg45,Ser46]-Eglin C kodierenden Gens

In analoger Weise wie in Beispiel 3 beschrieben wird die [Leu45,Asp46]→[Arg45,Ser46]-Mutation durchgeführt. Das verwendete mutagene Oligonucleotid hat folgende Struktur

```
                        *** **
        5'-GTA ACG CAG AGA ACG AGT AAC AGG-3'
```

Die Mutation des Eglin C Gens ist im folgenden Schema dargestellt.

```
    3'-CAT TGC GTC CAG GTC TCA TTG TCC-5'
                    *** **                          (I)
  + 5'-GTA ACG CAG AGA ACG AGT AAC AGG-3'

    (Oligo-primer)
              ↓
                    *** **
    5'-GTA ACG CAG AGA ACG AGT AAC AGG-3'
                                                    (II)
    3'-CAT TGC GTC CAG GTC TCA TTG TCC-5'

          ↓  Mutation


    5'-GTA ACG CAG AGA ACG AGT AAC AGG-3'
                                                    (III)
    3'-CAT TGC GTC TCT TGC TCA TTG TCC-5'
                47                    43
             Leu Ser Arg Thr Val
```

Bei der Aufarbeitung des Mutationsgemisches werden 12 potentielle [Arg45,Ser46]-Eglin C-Mutanten erhalten.

Durch Klonierung der [Arg45,Ser46]-Eglin C-DNA in den Vektor pHRi148/EcoRI/BamHI erhält man in analoger Weise wie im Beispiel 3 beschrieben das Plasmid pML147/b. Der mit diesem Plasmid transformierte E. coli HB101-Stamm wird als E. coli HB101/pML147/b bezeichnet.

### Beispiel 6: Kultivierung der transformierten E. coli-Stämme

Die transformierten E. coli HB101-Stämme werden über Nacht bei 37°C und 250 upm in 5 ml L-Medium (siehe Beispiel 2) kultiviert. 1 ml dieser Uebernachtkultur wird dann in 25 ml M9-Medium übertragen. M9-Medium ist wie folgt zusammengesetzt (pro 1 l):

| | |
|---|---|
| $Na_2HPO_4 \cdot 7H_2O$ | 13,25 g |
| $KH_2PO_4$ | 3,0 g |
| NaCl | 0,5 g |
| $NH_4Cl$ | 1,0 g |
| $CaCl_2 \cdot 2H_2O$ | 0,015 g |
| $MgSO_4 \cdot 7H_2O$ | 0,25 g |
| Casaminosäuren | 2,5 g |
| Vitamin $B_1$ | 0,0099 g |
| Glucose | 5,0 g |
| Ampicillin | 0,1 g |

Es wird bei 37°C und 250 upm kultiviert. Nach 8-10 Stunden hat die Kultur den höchsten Titer an Eglin C-Mutanten [bestimmt anhand der Messung der Protease Humanleukozyten-Elastase nach U. Seemüller et al., Hoppe-Seyler's Z. Physiol. Chem. 358, 1105 (1977)] erreicht.

### Beispiel 7: Isolierung und Reinigung der Eglin-Mutanten

Die überproduzierenden E. coli-Zellen werden mechanisch mit Hilfe einer Dyno-Mill aufgebrochen. Die Zelltrümmer werden in einer Sorval-Zentrifuge bei 9000 upm in 30 Minuten abzentrifugiert.

Auf Grund der hohen Stabilität der Eglin-Mutanten gegenüber Säuren kann der grösste Teil der Fremdproteine im Ueberstand durch Fällung mit ca. 20 % Essigsäure beseitigt werden: 10 ml 40 % wässrige Essigsäure werden tropfenweise innerhalb von 10 Minuten zu 100 ml Ueberstand pipettiert. Die saure Lösung (pH 3,4) wird 1 Stunde unter Eiskühlung gerührt. Die präzipitierten Fremdproteine und andere Zellbestandteile werden in einer Sorval-Zentrifuge bei 9000 upm in 30 Minuten abzentrifugiert. Der Ueberstand wird über Nacht in einem Virtis-Freezemobile lyophilisiert.

Das eingetrocknete gelbliche Lyophilisat wird in 10 ml 10 mM Tris-HCl pH 7,8 gelöst und zur Klärung der Lösung kurz zentrifugiert (Sorval SS34: 15000 upm, 10 Minuten). Der klare, gelbe Ueberstand wird auf eine äquilibrierte Sephadex G-50 superfine-Säule (Fa. Pharmacia) mit einer Länge von 100 cm und einem Durchmesser von 2,5 cm gegeben. Eluiert wird mit 10 mM Tris-HCl pH 7,8 und einer Flussrate von max. 20 ml/h. Die Absorption des Eluats bei 280 nm wird aufgezeichnet. Es werden Fraktionen à 10 ml gesammelt. Das Elutionsdiagramm zeigt einen hohen Peak (Fraktionen 31-40), welcher die jeweilige Eglin-Mutante enthält. Die Reinheit der Eglin-Mutanten in diesen Fraktionen wird durch SDS-Gelelektrophorese und HPLC bestätigt. Nach der Gelfiltration weisen die Eglin-Mutanten eine Reinheit von ca. 99 % auf.

Die Entfernung des Puffers aus den Eglin-Mutanten-Fraktionen erfolgt mit einer AMICON-Konzentrationszelle mit YM-2-Membran (MWCO 2000). Nach der Ultrafiltration werden die Proben erneut lyophilisiert. Man erhält ein farbloses Pulver (ca. 250 mg/100 ml Dyno-Mill-Aufschluss), das bei -20 °C gelagert wird.

Beispiel 8: Physikochemische Charakterisierung der Eglin-Mutanten

a. [Pro44]-Eglin C

Das gemäss Beispiel 7 gereinigte [Pro44]-Eglin C wird einer Molekulargewichtsbestimmung mittels FAB-MS unterworfen. Der Molekülion-Peak [M-H$^+$] wird bei 8130,6 ermittelt. Demnach handelt es sich bei dem verfahrensgemäss erhaltenen Produkt um N-Acetyl-[Pro44]-eglin C (theoretischer Wert für M-H$^+$: 8130,07).

Beim tryptischen Abbau der Eglin-Mutante ergeben sich 7 Fragmente, von denen sich nur das die Mutation Thr44→Pro44 enthaltene Fragment 4 von den entsprechenden Fragmenten von N$^\alpha$-Acetyl-eglin C (vgl. Europäische Patentanmeldung Nr. 146785) unterscheidet. Die Eglin-Mutante ist somit als N$^\alpha$-Acetyl-[Pro44]-eglin C zu bezeichnen.

In der PAGE-SDS-Gelelektrophorese [vgl. U.K. Laemmli, Nature 227, 680-685 (1970)] verhält sich N$^\alpha$-Acetyl-[Pro44]-eglin C wie N$^\alpha$-Acetyl-eglin C.

b. [Arg45]-Eglin C

Die Molekulargewichtsbestimmung des gereinigten [Arg45]-Eglin C ergibt einen Wert von 8175,4 [M-H$^+$]. Somit liegt auch hier eine N-Acetyl-Verbindung vor (theoretischer Wert für M-H$^+$: 8175). Der enzymatische Abbau mit Trypsin erbringt Klarheit, dass es sich um N$^\alpha$-Acetyl-[Arg45]-eglin C handelt.

In der PAGE-SDS-Gelelektrophorese verhält sich N$^\alpha$-Acetyl-[Arg45]-eglin C gleichfalls wie N$^\alpha$-Acetyl-eglin C.

c. [Arg45,Ser46]-Eglin C

Die Molekulargewichtsbestimmung des gereinigten [Arg45,Ser46]-Eglin C ergibt einen Wert von 8148,7 [M-H$^+$]. Somit liegt auch hier eine N-Acetyl-Verbindung vor (theoretischer Wert für M-H$^+$: 8149,1). Der tryptische Abbau der Eglin-Mutante ergibt, dass es sich um N$^\alpha$-Acetyl-[Arg45,Ser46]-eglin C handelt.

In der PAGE-SDS-Gelelektrophorese verhält sich N$^\alpha$-Acetyl-[Arg45,Ser46]-eglin C gleichfalls wie N$^\alpha$-Acetyl-eglin C.

Beispiel 9: Kinetische Charakterisierung der Eglin-Mutanten

Die Bestimmung der Inhibitionskonstanten $K_i$ erfolgt nach N. Braun et al. [Biol. Chem. Hoppe-Seyler 368, 299-308 (1987)] durch Messung der steady-state Reaktionsgeschwindigkeit des Freisetzens von p-Nitroanilin aus Proteinase-Inhibitor-Substrat-Mischungen. Nur die Inhibitor-Konzentration wird variiert. Die Freisetzung von p-Nitroanilin wird, nachdem die Kurve $OD_{405}$ vs. Zeit linear war, 10 bis 20 Minuten aufgezeichnet. Aus den verschiedenen Steigungen kann $K_i$ bestimmt werden. Als Proteinasen dienen Humanleukozyten-Elastase (HLE), Chymotrypsin und Trypsin. Exemplarische Inhibitionskonstanten sind in der folgenden Tabelle zusammmgestellt:

<u>Tabelle:</u> Inhibitionskonstanten

a. HLE:

|  | Eglin C | [Arg45]–Eglin C |
|---|---|---|
| $K_i$ (M) | $7,5 \times 10^{-11}$ | $1,1 \times 10^{-5}$ |

b. Chymotrypsin

|  |  |  |
|---|---|---|
| $K_i$ (M) | $4,4 \times 10^{-10}$ | $8 \times 10^{-9}$ |

c. Trypsin

|  |  |  |
|---|---|---|
| $K_i$ (M) | keine Inhibition | $1,3 \times 10^{-10}$ |

Die Ergebnisse zeigen, dass durch Austausch von Leu45 gegen Arg45 eine Eglin C-Mutante erhalten wird, welche im Gegensatz zu natürlichem Eglin C ein sehr starker Trypsin-Inhibitor aber nur noch ein schwacher HLE-Inhibitor ist.

<u>Beispiel 10:</u> <u>Expression von [Arg45]-Eglin C und N$^\alpha$-Acetyl-[Arg45]-eglin C in Hefe</u>

Ein Vektor für die Expression von Fremdgenen in Hefe erhält einen starken, vorzugsweise induzierbaren Hefe-Promotor und ein anschliessendes Transkriptions-Terminationssignal, welches mit dem Promotor durch singuläre Restriktionsstellen verbunden ist, die die Einfügung von Fremdgenen erlauben. Weiterhin erhält ein Hefe-Expressionsvektor DNA-Sequenzen, die die autonome Replikation des Vektors gewährleisten und zu einer hohen Kopienzahl führen. Eine solche Sequenz ist vorzugsweise die Hefe 2 $\mu$ DNA. Ferner enthält der Vektor einen selektierbaren Marker für Hefe, vorzugsweise das Hefe <u>LEU2</u> Gen, als auch pBR322 DNA-Sequenzen mit dem Replikationsstart und dem Ampicillin-Resistenzgen für die Amplifikation in <u>E. coli</u>. Solche Vektoren werden als "Schaukel"-Vektoren bezeichnet, da sie sowohl in Hefe als auch in <u>E. coli</u> verwendet werden können.

Ein entsprechender Expressionsvektor, der sehr effizient in Hefe eingesetzt werden kann, wurde in der Europäischen Patentanmeldung Nr. 100561 beschrieben. Die Expression von Fremdgenen erfolgt unter der Kontrolle des regulierbaren <u>PHO5</u> Promotors des sauren Phosphatase-Gens der Hefe. Der <u>PHO5</u> Promotor, das Fremdgen und die <u>PHO5</u> Transkriptions-Terminationssignal-Sequenzen wurden hintereinander geschaltet in das Plasmid pJDB207 integriert, welches die Hefe 2$\mu$ DNA, das Hefe <u>LEU2</u> Gen, einen <u>E. coli</u> Replikationsstart sowie das Ampicillin-Resistenzgen enthält.

Das Expressionsplasmid pJDB207R/<u>PHO5</u>-[Arg45]EGL wird wie folgt konstruiert:

a) <u>Isolierung des pJDB207 Vektor-Fragmentes</u>

Sechs $\mu$g des Plasmids pJDB207R/IF($\alpha$-3) (EP 100561) werden mit dem Restriktionsenzym BamHI vollständig verdaut. Die entstandenen DNA-Fragmente von 6,85 kb und 1,15 kb Grösse werden mit Ethanol präzipitiert und in 400 $\mu$l 50 mM Tris-HCl, pH 8,0 resuspendiert. 4,5 Einheiten alkalischer Phosphatase (aus Kälberdärmen, Boehringer Mannheim) werden zugegeben, und das Gemisch wird für 1 Stunde bei 37°C inkubiert. Anschliessend wird die Phosphatase durch 1,5 stündige Inkubation bei 65°C inaktiviert. Die Lösung wird auf eine Konzentration von 150 mM NaCl eingestellt und anschliessend über eine 100 $\mu$l DE 52 (Whatman) Anionenaustauschersäule gegeben, die mit einer Lösung von 10 mM Tris-HCl, pH 7,5, 150 mM NaCl, 1 mM EDTA equilibriert wurde. Nach Waschen mit dem gleichen Puffer wird die DNA mit 400 $\mu$l 10 mM Tris-HCl, pH 7,5, 1,5 M NaCl, 1 mM EDTA eluiert und mit Ethanol präzipitiert. Das 6,85 kb grosse BamHI-Fragment wird von dem kleinen Fragment in einem 0,6%-igen Gel aus niedrigschmelzender Agarose in Tris-Borat-EDTA Puffer, pH 8,3, abgetrennt.

b) <u>Isolierung des 534 bp grossen PHO5 Promotor-Fragmentes</u>

Zehn $\mu$g des Plasmids p31/R (EP 100561) werden mit den Restriktionsenzymen EcoRI und BamHI verdaut. Die entstandenen 3 Fragmente werden in einem 0,6%-igen Gel aus niedrigschmelzender Agarose in Tris-Borat-EDTA Puffer, pH 8,3, getrennt. Das 534 bp grosse BamHI-EcoRI Fragment, welches den PHO5 Promotor sowie den Transkriptionsstart enthält, wird isoliert.

c) Isolierung des 230 bp grossen DNA-Fragmentes, welches die kodierende Sequenz für [Arg45]-Eglin C enthält

Acht μg des Plasmids pJB618 werden mit den Restriktionsenzymen BamHI und EcoRI verdaut. Die entstandenen 2 Fragmente werden in einem 0,6%-igen Gel aus niedrigschmelzender Agarose in Tris-Borat-EDTA Puffer, pH 8,3, getrennt. Das 230 bp grosse Fragment wird isoliert.

d) Ligation der DNA Fragmente

Die drei unter a) - c) beschriebenen DNA-Fragmente, die einander entsprechende überhängende Enden besitzen, werden in einer Ligationsreaktion miteinander verknüpft. 0,1 pmol (0,45 μg) des 6,85 kb grossen BamHI Vektor-Fragmentes, 0,2 pmol (70 ng) des 543 bp grossen BamHI-EcoRI PHO5 Promotor-Fragmentes und 0,2 pmol (29 ng) des 230 bp grossen EcoRI-BamHI-Fragmentes von pJB618 werden ligiert. Alle drei DNA-Fragmente sind in kleinen Blöcken niedrigschmelzender Agarose enthalten. Die drei Agarose-Blöcke werden vereinigt, bei 65°C geschmolzen und zweifach verdünnt. Die Ligation wird in einem Endvolumen von 270 μl in 60 mM Tris-HCl, pH 7,5, 10 mM MgCl$_2$, 10 mM DTT, 1 mM ATP mit 16 Einheiten T4 DNA-Ligase (Boehringer Mannheim) bei 15°C während 16 Stunden durchgeführt. 10 μl des Ligationsgemisches werden zu 100 μl Calcium-behandelter, kompetenter E. coli HB101 Zellen gegeben.

24 transformierte, Ampicillin-resistente Einzelkolonien werden jeweils in LB-Medium, welches 100 μg/ml Ampicillin enthält, kultiviert. Die Plasmid-DNA wird nach Holmes et al. [Anal. Biochem. 114, 193 (1981)] isoliert und durch HindIII/EcoRI Doppelrestriktion analysiert. Das Auftreten eines 600 bp grossen EcoRI-HindIII-Fragmentes bezeichnet diejenigen Klone, welche das PHO5 Promotor-[Arg45]-Eglin C-DNA-Fragment in der richtigen Orientierung im Expressionsvektor integriert enthalten. Wie erwartet, enthalten 50 % der Klone das Insert in der richtigen Orientierung. Einer dieser Klone wird isoliert und als pJDB207R/PHO5-[Arg45]EGL bezeichnet.

e) Transformation von Saccharomyces cerevisiae Stamm GRF18

Nach dem Transformationsprotokoll von Hinnen et al. [Proc. Natl. Acad. Sci. USA 75, 1929 (1978)] wird das Plasmid pJDB207R/PHO5-[Arg45]EGL in Zellen von Saccharomyces cerevisiae Stamm GRF18 (α, his3-11, his3-15, leu2-3, leu2-112, can$^R$) transformiert. Transformierte Zellen werden auf Hefe Minimalmedium-Platten, die kein Leucin enthalten, selektioniert. Einzelne transformierte Hefekolonien werden isoliert und als Saccharomyces cerevisiae GRF18/pJDB207R/PHO5-[Arg45]EGL bezeichnet.

f) Fermentation von Saccharomyces cerevisiae GRF18/pJDB207R/PHO5-[Arg45]EGL und Isolierung von [Arg45]-Eglin C und N$^\alpha$-Acetyl-[Arg45]-eglin C

Saccharomyces cerevisiae GRF18/pJDB207R/PHO5-[Arg45]EGL Zellen werden in 3 l Minimalmedium mit 0,03 g/l KH$_2$PO$_4$ in einem Mini-Bioreaktor bei 30°C kultiviert und bei Erreichen einer Zelldichte, die einer OD$_{660}$ von 1,9 entspricht, geerntet.

[Arg45]-Eglin C und N$^\alpha$-Acetyl-[Arg45]-eglin C werden von den transformierten Hefezellen im Gewichtsverhältnis von etwa 2:1 gebildet. Beide Produkte können aus Hefezellhomogenaten entsprechend der in Beispiel 7 für E. coli angegebenen Methode isoliert werden.

Beispiel 11: Pharmazeutisches Präparat

Eine gemäss Beispiel 7 hergestellte, N$^\alpha$-Acetyl-[Arg45]-eglin C enthaltende Lösung wird gegen eine 0,9%-ige NaCl-Lösung dialysiert. Die Konzentration der Lösung wird danach durch Verdünnen mit der gleichen NaCl-Lösung auf 1 mg/ml oder 10 mg/ml eingestellt. Diese Lösungen werden durch Ultrafiltration (Membranen mit 0,22 μm Poren) sterilisiert.

Die sterilisierten Lösungen sind direkt verwendbar für die intravenöse Verabreichung und für die Dauertropfinfusion.

Hinterlegung von Mikroorganismen

Der Stamm E. coli HB101/pML147 wurde am 28. Januar 1988 bei der Deutschen Sammlung von Mikroorganismen (DSM), Mascheroder Weg 1b, D-3300 Braunschweig, unter der Nummer DSM 4380 hinterlegt.

**Patentansprüche**
**Patentansprüche für folgende Vertragsstaaten : AT, BE, CH, DE, FR, GB, IT, LI, LU, NL, SE**

1. Verbindungen der Formel

```
R-ThrGluPheGlySerGluLeuLysSerPheProGluValValGlyLysThrVal

AspGlnAlaArgGluTyrPheThrLeuHisTyrProGlnTyrAspVal-W-PheLeuProGluGly

SerProVal-X-Y-Z-LeuArgTyrAsnArgValArgValPheTyrAsnProGlyThrAsnVal

ValAsnHisValProHisValGly-OH        (I),
```

worin R für Wasserstoff oder Acetyl steht, W Tyr oder His bedeutet, X Thr, Ser oder Pro ist, Y für Leu, Met, Lys, Phe, Tyr oder Trp steht und Z Asp, Glu, Gln, Asn, Ala, Ser oder Thr ist, mit der Massgabe, dass X von Thr verschieden ist, wenn Y für Leu steht und Z Asp ist, und Salze davon.

2. Verbindungen der Formel I gemäss Anspruch 1, worin R Acetyl ist, W Tyr bedeutet und entweder X Thr ist, Y Arg oder Lys ist und Z Asp, Glu, Gln, Asn, Ala, Ser oder Thr ist, oder X Pro ist, Y Met ist und Z Asp ist, oder X Thr ist, Y Phe, Tyr, Trp oder Met ist und Z Asp ist, und Salze davon.

3. Verbindungen der Formel I gemäss Anspruch 1, worin R Acetyl ist, W Tyr bedeutet, X Thr ist, Y für Arg steht und Z Asp oder Ser ist, und Salze davon.

4. N$^\alpha$-Acetyl-[Arg45]-eglin C gemäss Anspruch 1.

5. Pharmazeutische Zusammensetzung enthaltend eine Verbindung der Formel I gemäss Anspruch 1 oder ein pharmazeutisch annehmbares Salz davon.

6. Eine Verbindung der Formel I gemäss Anspruch 1 oder ein pharmazeutisch annehmbares Salz davon zur Anwendung in einem Verfahren zur prophylaktischen oder therapeutischen Behandlung des menschlichen und tierischen Körpers.

7. Verfahren zur Herstellung von Verbindungen der Formel I gemäss Anspruch 1 und ihrer Salze, dadurch gekennzeichnet, dass man einen transformierten Wirtsmikroorganismus, der eine DNA enthält, welche für eine Verbindung der Formel I kodiert, kultiviert und die Verbindung der Formel I oder ein Salz davon isoliert.

8. Eine DNA, welche eine für eine Verbindung der Formel I gemäss Anspruch 1 kodierende DNA-Sequenz aufweist.

9. Ein Expressionsvektor, welcher eine für eine Verbindung der Formel I gemäss Anspruch 1 kodierende und von einer Expressionskontrollsequenz kontrollierte DNA-Sequenz enthält.

10. Ein Wirtsmikroorganismus enthaltend einen Expressionsvektor gemäss Anspruch 9.

11. Eine nach dem Verfahren gemäss Anspruch 7 erhältliche Verbindung.

12. Verfahren zur Herstellung einer DNA, welche eine für eine Verbindung der Formel I gemäss Anspruch 1 kodierende DNA-Sequenz aufweist, dadurch gekennzeichnet, dass man die DNA durch chemische Synthese herstellt oder Fragmente davon durch chemische Synthese herstellt und diese in vorherbestimmter Weise enzymatisch verknüpft, oder dass man eine für Eglin B oder Eglin C kodierende DNA

16

in einem oder mehreren Schritten mutiert.

13. Verfahren zur Herstellung eines Expressionsvektors, welcher eine für eine Verbindung der Formel I gemäss Anspruch 1 kodierende und von einer Expressionskontrollsequenz kontrollierte DNA-Sequenz enthält, dadurch gekennzeichnet, dass man in eine Vektor-DNA, welche eine Expressionskontrollsequenz enthält, eine für die Verbindung der Formel I kodierende DNA-Sequenz derart einfügt, dass die Expressionskontrollsequenz besagte DNA-Sequenz reguliert.

14. Verfahren zur Herstellung eines Wirtsmikroorganismus, welcher einen nach dem Verfahren gemäss Anspruch 13 erhältlichen Expressionsvektor enthält, dadurch gekennzeichnet, dass man eine Wirtszelle mit einem Expressionsvektor, der eine von einer Expressionskontrollsequenz regulierte, für eine Verbindung der Formel I kodierende DNA-Sequenz enthält, transformiert.

**Patentansprüche für folgende Vertragsstaaten : ES, GR**

1. Verfahren zur Herstellung von Verbindungen der Formel

```
R-ThrGluPheGlySerGluLeuLysSerPheProGluValValGlyLysThrVal

AspGlnAlaArgGluTyrPheThrLeuHisTyrProGlnTyrAspVal-W-PheLeuProGluGly

SerProVal-X-Y-Z-LeuArgTyrAsnArgValArgValPheTyrAsnProGlyThrAsnVal

ValAsnHisValProHisValGly-OH        (I),
```

worin R für Wasserstoff oder Acetyl steht, W Tyr oder His bedeutet, X Thr, Ser oder Pro ist, Y für Leu, Met, Arg, Lys, Phe, Tyr oder Trp steht und Z Asp, Glu, Gln, Asn, Ala, Ser oder Thr ist, mit der Massgabe, dass X von Thr verschieden ist, wenn Y für Leu steht und Z Asp ist, und Salzen davon, dadurch gekennzeichnet, dass man einen transformierten Wirtsmikroorganismus, der eine DNA enthält, welche für eine Verbindung der Formel I kodiert, kultiviert und die Verbindung der Formel I oder ein Salz davon isoliert.

2. Verfahren zur Herstellung von Verbindungen der Formel I gemäss Anspruch 1, worin R Acetyl ist, W Tyr bedeutet und entweder X Thr ist, Y Arg oder Lys ist und Z Asp, Glu, Gln, Asn, Ala, Ser oder Thr ist, oder X Pro ist, Y Met ist und Z Asp ist, oder X Thr ist, Y Phe, Tyr, Trp oder Met ist und Z Asp ist, und Salzen davon.

3. Verfahren zur Herstellung von Verbindungen der Formel I gemäss Anspruch 1, worin R Acetyl ist, W Tyr bedeutet, X Thr ist, Y für Arg steht und Z Asp oder Ser ist, und Salzen davon.

4. Verfahren zur Herstellung von N$^\alpha$-Acetyl-[Arg45]-eglin C gemäss Anspruch 1.

5. Verfahren zur Herstellung von pharmazeutischen Zusammensetzungen, dadurch gekennzeichnet, dass man eine gemäss Anspruch 1 herstellbare Verbindung der Formel I oder ein pharmazeutisch annehmbares Salz davon mit einem konventionellen pharmazeutisch annehmbaren Träger und/oder Hilfsstoffen mischt.

6. Verfahren zur Herstellung einer DNA, welche eine für eine gemäss Anspruch 1 herstellbare Verbindung der Formel I kodierende DNA-Sequenz aufweist, dadurch gekennzeichnet, dass man die DNA durch chemische Synthese herstellt oder Fragmente davon durch chemische Synthese herstellt und diese in vorherbestimmter Weise enzymatisch verknüpft, oder dass man eine für Eglin B oder Eglin C kodierende DNA in einem oder mehreren Schritten mutiert.

7. Verfahren zur Herstellung eines Expressionsvektors, welcher eine für eine gemäss Anspruch 1 herstellbare Verbindung der Formel I kodierende und von einer Expressionskontrollsequenz kontrollierte DNA-Sequenz enthält, dadurch gekennzeichnet, dass man in eine Vektor-DNA, welche eine Expressionskontrollsequenz enthält, eine für die Verbindung der Formel I kodierende DNA-Sequenz derart einfügt, dass die Expressionskontrollsequenz besagte DNA-Sequenz reguliert.

**8.** Verfahren zur Herstellung eines Wirtsmikroorganismus, welcher einen nach dem Verfahren gemäss Anspruch 7 erhältlichen Expressionsvektor enthält, dadurch gekennzeichnet, dass man eine Wirtszelle mit einem Expressionsvektor, der eine von einer Expressionskontrollsequenz regulierte, für eine Verbindung der Formel I kodierende DNA-Sequenz enthält, transformiert.

**9.** Ein Wirtsmikroorganismus enthaltend einen Expressionsvektor herstellbar gemäss Anspruch 7.

**Claims**
**Claims for the following Contracting States : AT, BE, CH, DE, FR, GB, IT, LI, LU, NL, SE**

**1.** A compound of formula

```
R-ThrGluPheGlySerGluLeuLysSerPheProGluValValGlyLysThrVal
AspGlnAlaArgGluTyrPheThrLeuHisTyrProGlnTyrAspVal-W-PheLeu
ProGluGlySerProVal-X-Y-Z-LeuArgTyrAsnArgValArgValPheTyr
AsnProGlyThrAsnValValAsnHisValProHisValGly-OH     (I),
```

in which
R is hydrogen or acetyl,
W is Tyr or His,
X is Thr, Ser or Pro,
Y is Leu, Met, Arg, Lys, Phe, Tyr or Trp, and
Z is Asp, Glu, Gln, Asn, Ala, Ser or Thr,
with the proviso that X is other than Thr when Y is Leu and Z is Asp, or a salt thereof.

**2.** A compound of formula I according to claim 1 in which
R is acetyl,
W is Tyr and either
X is Thr, Y is Arg or Lys and Z is Asp, Glu, Gln, Asn, Ala, Ser or Thr, or
X is Pro, Y is Met and Z is Asp, or
X is Thr, Y is Phe, Tyr, Trp or Met and Z is Asp,
or a salt thereof.

**3.** A compound of formula I according to claim 1 in which
R is acetyl,
W is Tyr,
X is Thr,
Y is Arg and
Z is Asp or Ser,
or a salt thereof.

**4.** $N^{\alpha}$-Acetyl-[Arg$^{45}$]-eglin C according to claim 1.

**5.** A pharmaceutical composition comprising a compound of formula I according to claim 1 or a pharmaceutically acceptable salt thereof.

**6.** A compound of formula I according to claim 1 or a pharmaceutically acceptable salt thereof for use in a method for the prophylactic or therapeutic treatment of the human or animal body.

**7.** A process for the preparation of a compound of formula I according to claim 1 or a salt thereof, which comprises culturing a transformed host microorganism comprising a DNA that codes for a compound of formula I and isolating therefrom the compound of formula I or a salt thereof.

**8.** A DNA comprising a DNA sequence that codes for a compound of formula I according to claim 1.

9. An expression vector comprising a DNA sequence that codes for a compound of formula I according to claim 1 and that is controlled by an expression control sequence.

10. A host microorganism comprising an expression vector according to claim 9.

11. A compound obtainable in accordance with the process of claim 7.

12. A process for the production of a DNA that comprises a DNA sequence coding for a compound of formula I according to claim 1, which comprises producing the DNA by chemical synthesis or producing fragments thereof by chemical synthesis and linking these enzymatically in a predetermined manner, or mutating a DNA coding for eglin B or eglin C in one or more steps.

13. A process for the production of an expression vector comprising a DNA sequence that codes for a compound of formula I according to claim 1 and is controlled by an expression control sequence, which comprises so inserting a DNA sequence that codes for a compound of formula I into a vector DNA that comprises an expression control sequence that the expression control sequence regulates the said DNA sequence.

14. A process for the production of a host microorganism that comprises an expression vector obtainable in accordance with the process of claim 13, which comprises transforming a host cell using an expression vector comprising a DNA sequence that codes for a compound of formula I and is regulated by an expression control sequence.

**Claims for the following Contracting States : ES, GR**

1. A process for the preparation of a compound of formula

```
R-ThrGluPheGlySerGluLeuLysSerPheProGluValValGlyLysThrVal
AspGlnAlaArgGluTyrPheThrLeuHisTyrProGlnTyrAspVal-W-PheLeu
ProGluGlySerProVal-X-Y-Z-LeuArgTyrAsnArgValArgValPheTyr
AsnProGlyThrAsnValValAsnHisValProHisValGly-OH     (I),
```

in which
R is hydrogen or acetyl,
W is Tyr or His,
X is Thr, Ser or Pro,
Y is Leu, Met, Arg, Lys, Phe, Tyr or Trp, and
Z is Asp, Glu, Gln, Asn, Ala, Ser or Thr,
with the proviso that X is other than Thr when Y is Leu and Z is Asp, or a salt thereof, which comprises culturing a transformed host microorganism comprising a DNA that codes for a compound of formula I and isolating therefrom the compound of formula I or a salt thereof.

2. A process for the preparation of a compound of formula I according to claim 1, in which
R is acetyl,
W is Tyr and either
X is Thr, Y is Arg or Lys and Z is Asp, Glu, Gln, Asn, Ala, Ser or Thr, or
X is Pro, Y is Met and Z is Asp, or
X is Thr, Y is Phe, Tyr, Trp or Met and Z is Asp,
or a salt thereof.

3. A process for the preparation of a compound of formula I according to claim 1, in which
R is acetyl,
W is Tyr,
X is Thr,

Y is Arg and
Z is Asp or Ser,
or a salt thereof.

4. A process for the preparation of N$^\alpha$-acetyl-[Arg$^{45}$]-eglin C according to claim 1.

5. A process for the preparation of a pharmaceutical composition which comprises mixing a compound of formula I obtainable in accordance with claim 1 or a pharmaceutically acceptable salt thereof with a conventional pharmaceutically acceptable carrier and/or with excipients.

6. A process for the production of a DNA that comprises a DNA sequence coding for a compound of formula I obtainable in accordance with claim 1, which comprises producing the DNA by chemical synthesis or producing fragments thereof by chemical synthesis and linking these enzymatically in a predetermined manner, or mutating a DNA coding for eglin B or eglin C in one or more steps.

7. A process for the production of an expression vector comprising a DNA sequence that codes for a compound of formula I obtainable in accordance with claim 1 and is controlled by an expression control sequence, which comprises so inserting a DNA sequence that codes for a compound of formula I into a vector DNA that comprises an expression control sequence that the expression control sequence regulates the said DNA sequence.

8. A process for the production of a host microorganism that comprises an expression vector obtainable in accordance with the process of claim 7, which comprises transforming a host cell using an expression vector comprising a DNA sequence that codes for a compound of formula I and is regulated by an expression control sequence.

9. A host microorganism that comprises an expression vector obtainable in accordance with claim 7.

**Revendications**
**Revendications pour les Etats contractants suivants : AT, BE, CH, DE, FR, GB, IT, LI, LU, NL, SE**

1. Composés de formule

```
R-ThrGluPheGlySerGluLeuLysSerPheProGluValValGlyLysThrVal

AspGlnAlaArgGluTyrPheThrLeuHisTyrProGlnTyrAspVal-W-PheLeuProGluGly

SerProVal-X-Y-Z-LeuArgTyrAsnArgValArgValPheTyrAsnProGlyThrAsnVal

ValAsnHisValProHisValGly-OH        (I),
```

où R représente l'hydrogène ou l'acétyle, W représente Tyr ou His, X est Thr, Ser ou Pro, Y représente Leu, Met, Arg, Lys, Phe, Tyr ou Trp et Z est Asp, Glu, Gln, Asn, Ala, Ser ou Thr, sous réserve que X est différent de Thr, lorsque Y représente Leu et Z est Asp et leurs sels.

2. Composés de formule I selon la revendication 1, où R est l'acétyle, W représente Tyr et soit X est Thr, Y est Arg ou Lys et Z est Asp, Glu, Gln, Asn, Ala, Ser ou Thr, soit X est Pro, Y est Met et Z est Asp, soit X est Thr, Y est Phe, Tyr, Trp, ou Met et Z est Asp et leurs sels.

3. Composés de formule I selon la revendication 1,où R est l'acétyle, W représente Tyr, X est Thr, Y représente Arg et Z est Asp ou Ser et leurs sels.

4. L'égline C N$^\alpha$-acétyle [Arg$^{45}$] selon la revendication 1.

5. Composition pharmaceutique contenant un composé de formule I selon la revendication 1 ou l'un de ses sels pharmaceutiquement acceptables.

6. Composés de formule I selon la revendication 1 ou l'un de ses sels pharmaceutiquement acceptables destiné à être utilisé dans un procédé pour le traitement prophylactique ou thérapeutique du corps humain et animal.

7. Procédé pour la préparation des composés de formule I selon la revendication 1 et de leurs sels, caractérisé en ce que l'on cultive un microorganisme hôte transformé, contenant un ADN codant pour un composé de formule I et en ce que l'on isole le composé de formule I ou l'un de ses sels.

8. Un ADN présentant une séquence d'ADN codant pour un composé de formule I selon la revendication 1.

9. Un vecteur d'expression contenant une séquence d'ADN codant pour un composé de formule I selon la revendication 1 et contrôlée par une séquence de contrôle de l'expression.

10. Un microorganisme hôte contenant un vecteur d'expression selon la revendication 9.

11. Un composé obtenu par le procédé selon la revendication 7.

12. Procédé pour la préparation d'un ADN présentant une séquence d'ADN codant pour un composé de formule I selon la revendication 1, caractérisé ence que l'on prépare l'ADN par synthèse chimique ouen ce que l'on prépare des fragments de celui-ci par synthèse chimique et en ce que l'on assemble ceux-ci par voie enzymatique de façon prédéterminée ou en ce que l'on mute un ADN codant pour l'égline B ou l'égline C en une ou plusieurs étapes.

13. Procédé pour la préparation d'un vecteur d'expression contenant une séquence d'ADN codant pour un composé de formule I selon la revendication 1 et contrôlée par une séquence de contrôle de l'expression, caractérisé en ce que l'on insère dans un ADN vecteur contenant une séquence de contrôle de l'expression une séquence d'ADN codant pour le composé de formule I de telle façon que la séquence de contrôle de l'expression régule ladite séquence d'ADN.

14. Procédé pour la préparation d'un microorganisme hôte contenant un vecteur d'expression obtenu par le procédé selon la revendication 13, caractérisé en ce que l'on transforme une cellule hôte avec un vecteur d'expression contenant une séquence d'ADN régulée par une séquence de contrôle de l'expression et codant pour un composé de formule I.

**Revendications pour les Etats contractants suivants : ES, GR**

1. Procédé pour la préparation des composés de formule

```
R-ThrGluPheGlySerGluLeuLysSerPheProGluValValGlyLysThrVal
AspGlnAlaArgGluTyrPheThrLeuHisTyrProGlnTyrAspVal-W-PheLeuProGluGly
SerProVal-X-Y-Z-LeuArgTyrAsnArgValArgValPheTyrAsnProGlyThrAsnVal
ValAsnHisValProHisValGly-OH          (I),
```

où R représente l'hydrogène ou l'acétyle, W représente Tyr ou His, X est Thr, Ser ou Pro, Y représente Leu, Met, Arg, Lys, Phe, Tyr ou Trp et Z est Asp, Glu, Gln, Asn, Ala, Ser ou Thr, sous réserve que X est différent de Thr, lorsque Y représente Leu et Z est Asp et de leurs sels, caractérisé en ce que l'on cultive un microorganisme hôte transformé contenant un ADN codant pour un composé de formule I et en ce que l'on isole le composé de formule I ou l'un de ses sels.

2. Procédé pour la préparation des composés de formule I selon la revendication 1 où R est l'acétyle, W représente Tyr et soit X est Thr, Y est Arg ou Lys et Z est Asp, Glu, Gln, Asn, Ala, Ser ou Thr, soit X est Pro, Y est Met et Z est Asp, soit X est Thr, Y est Phe , Tyr, Trp ou Met et Z est Asp et de leurs sels.

**3.** Procédé pour la préparation des composés de formule I selon la revendication 1, où R est l'acétyle, W représente Tyr, X est Thr, Y représente Arg et Z est Asp ou Ser et de leurs sels.

**4.** Procédé pour la préparation de l'égline C N$^\alpha$-acétyle [Arg$^{45}$] selon la revendication 1.

**5.** Procédé pour la préparation de compositions pharmaceutiques, caractérisé en ce que l'on mélange un composé de formule I pouvant être préparé selon la revendication 1 ou l'un de ses els pharmaceutiquement acceptables avec un support classique pharmaceutiquement acceptable et/ou des adjuvants.

**6.** Procédé pour la préparation d'un ADN présentant une séquence d'ADN codant pour un composé de formule I pouvant être préparé selon la revendication 1, caractérisé en ce l'on prépare l'ADN par synthèse chimique ou en ce que l'on prépare des fragments de celui-ci par synthèse chimique et en ce que l'on assemble ceux-ci par voie enzymatique de façon prédéterminée ou en ce que l'on mute un ADN codant pour l'égline B ou l'égline C en une ou plusieurs étapes.

**7.** Procédé pour la préparation d'un vecteur d'expression contenant une séquence d'ADN codant pour un composé de formule I pouvant être préparé selon la revendication 1 et controlée par une séquence de contrôle de l'expression, caractérisée en ce que l'on insère dans un ADN vecteur contenant une séquence de contrôle de l'expression une séquence d'ADN codant pour le composé de formule I, de telle façon que la séquence de contrôle de l'expression régule ladite séquence d'ADN.

**8.** Procédé pour la préparation d'un microorganisme hôte contenant un vecteur d'expression obtenu par le procédé selon la revendication 7, caractérisé en ce que l'on transforme une cellule hôte avec un vecteur d'expression contenant une séquence d'ADN régulée par une séquence de contrôle de l'expression et codant pour un composé de formule I.

**9.** Un microoorganisme hôte contenant un vecteur d'expression pouvant être préparé selon la revendication 7.